# EUROPEAN PATENT APPLICATION

(11) **EP 4 253 368 A1**
(43) Date of publication of application: **04.10.2023**
(21) Application number: 23164528.4
(22) Date of filing: 28.03.2023
(51) Int. Cl.: C07D 251/54

(54) **PROCESS FOR THE PREPARATION OF TAUTOMERIC FORMS OF SUNSCREENS**

(30) Priority: 30.03.2022 IT 202200006299
(71) Applicant: 3V SIGMA S.p.A, 20121 Milano (IT)
(72) Inventor: CAVALLINI, Giovanni, 20121 Milano (IT)
(74) Representative: Bianchetti & Minoja with Trevisan & Cuonzo IPS SRL

(57) **Abstract**

The compound of Formula 1 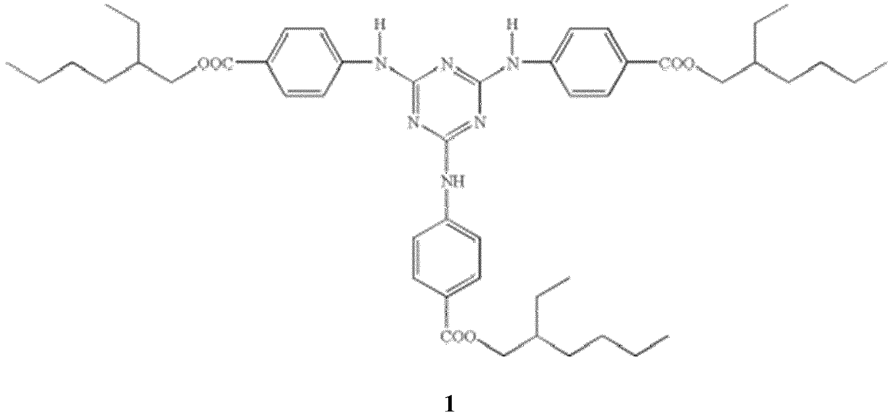 is obtained efficiently and cheaply from the mixture of tautomeric forms by crystallisation from methanol.

## Description

The object of the present invention is a process for the preparation of the tautomer of Formula 1 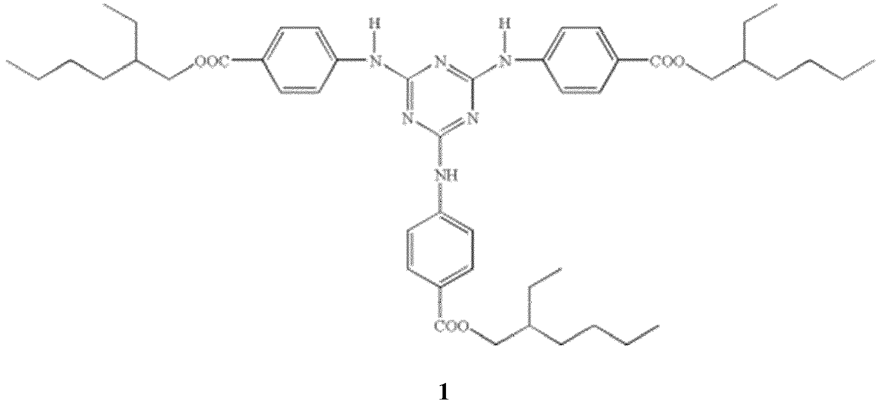 by crystallisation of mixtures of tautomers from methanol.

### Prior art

The compound 2, 4, 6-trianilino-p-(carbo-2-ethylhexyl-1'-oxy)-1, 3, 5-triazine (CAS number 88122-99-0), disclosed in EP 87098, is a well-known sunscreen used in cosmetic compositions marketed in Europe and the USA.

Said compound can exist in various tautomeric forms, one of which, disclosed in EP 1483250 and represented by Formula 1 below, is particularly effective and convenient for the preparation of oils and creams designed to protect the skin against the adverse effects of UVA and UVB solar radiation.

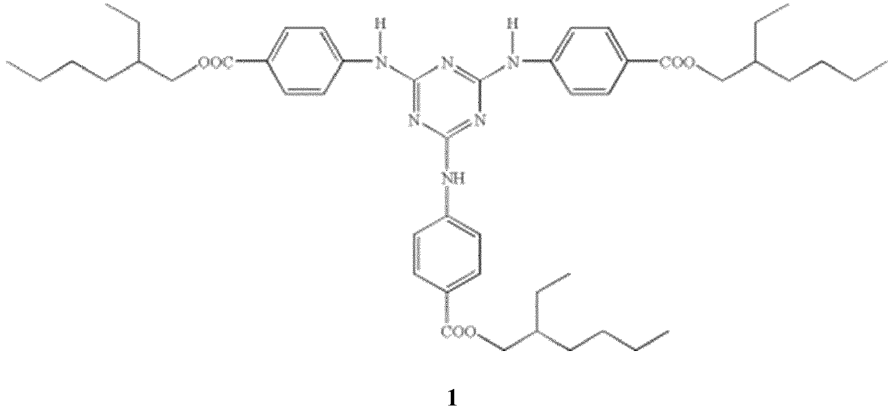

EP 1483250 discloses a system of crystallisation of the tautomer mixture from solvents or mixtures of C2-C8 alcohols, esters, carbonates, ketones and carbonitriles, and in particular ethyl alcohol.

### Description of the invention

It has now surprisingly been found that the compound of Formula 1 can be obtained by crystallising from methanol the tautomer mixture obtainable by reacting cyanuryl chloride with 2-ethylhexyl p-aminobenzoate.

Methanol is more convenient to use than ethanol for reasons of cost and legislative constraints, as ethanol is subject to specific tax controls which complicate its use for industrial purposes.

The object of the invention is therefore a process for the preparation of the compound of Formula 1 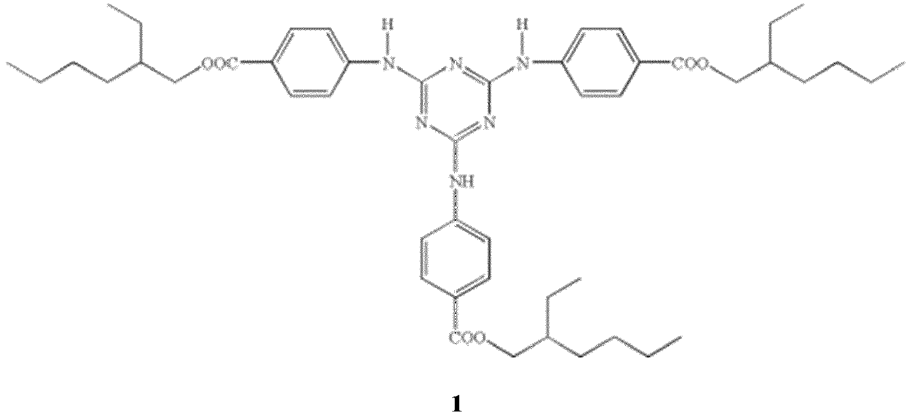 comprising crystallisation from methanol of the tautomer mixture obtained by reacting cyanuryl chloride with 2-ethylhexyl p-aminobenzoate.

The tautomer mixture obtained by reacting cyanuryl chloride with 2-ethylhexyl p-aminobenzoate, the reaction typically being conducted in xylene at reflux, can be discharged from the reactor in the molten state at a temperature of 100-120°C. After cooling, the molten mixture can be added gradually to methanol, preferably at boiling point. Alternatively, the molten mixture can be cooled and ground to any particle size.

The weight ratio between the tautomer mixture and methanol preferably ranges between 25% and 35%, more preferably between 27% and 31%. The methanol solution can be filtered before cooling (to about 15°C).

The invention is illustrated in greater detail in the examples below.

### Example 1

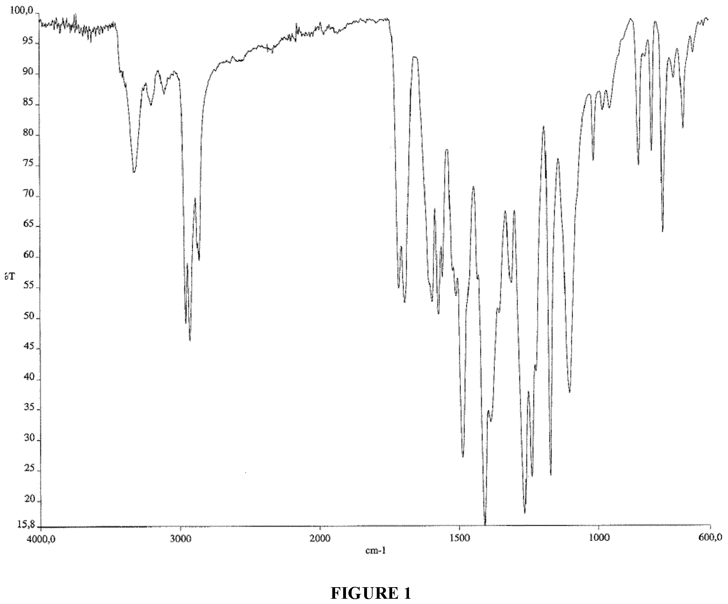

900 ml of xylol, 22.5 g of cyanuryl chloride and 94.2 g of p-aminobenzoic acid 2-ethylhexyl ester are loaded into a laboratory reactor. The mixture is heated at boiling point for about six hours, and the hydrochloric acid that develops is collected in a dilute soda solution. At the end of the reaction the xylol is distilled, but the reactor is maintained at a temperature of not less than 110/120°C, to obtain a tautomer mixture of Formula 1 in the molten state having the IR spectrum shown in Figure 1.

### Example 2

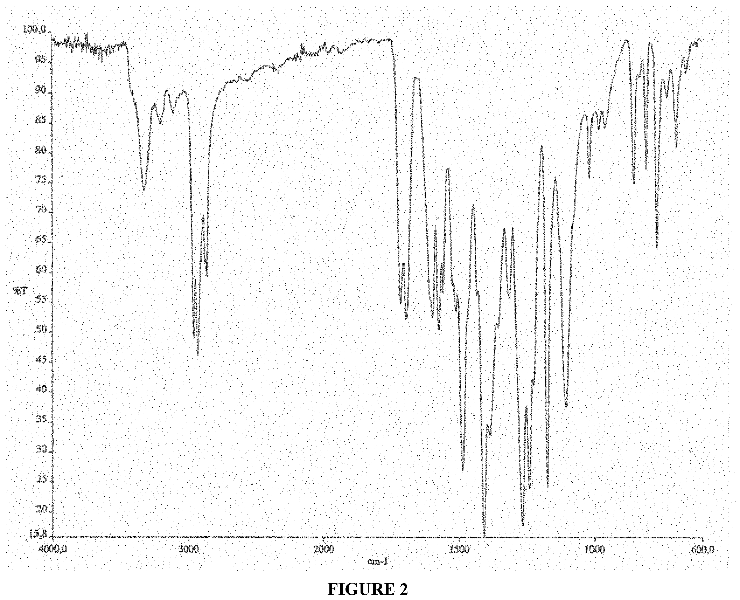

500 ml of benzine with a boiling point higher than 140°C, 11 g of cyanuryl chloride and 50 g of p-aminobenzoic acid 2-ethylhexyl ester are loaded into a laboratory reactor. The mixture is heated to boiling point for about eight hours, and the hydrochloric acid that develops is collected in a dilute soda solution. At the end of the reaction the solvent is distilled, and the reactor is maintained at a temperature of not less than 110/120°C, to obtain a tautomer mixture of Formula 1 in the molten state. The molten mixture is discharged into a flat container and left to cool until solid. The resulting solid product is then ground. The IR spectrum is shown in Figure 2.

### Example 3

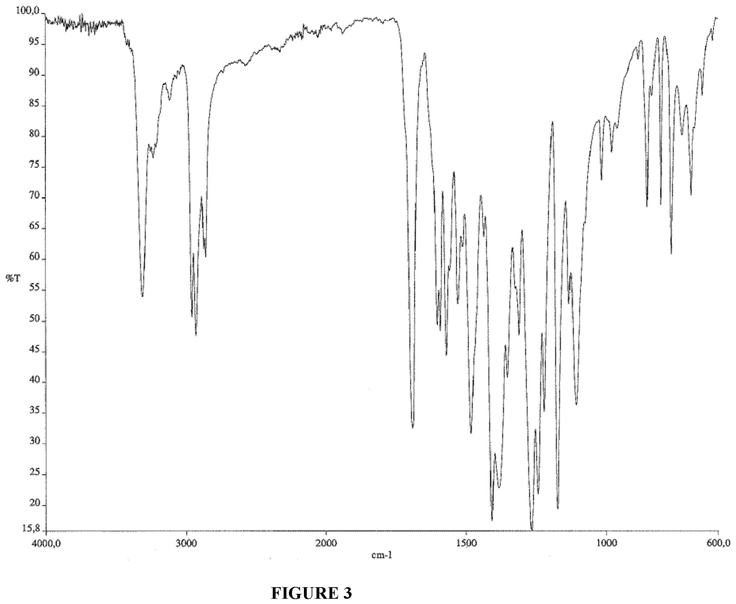

600 ml of methyl alcohol is loaded into a laboratory reactor and heated to gentle boiling. The tautomer mixture prepared in Example 1 is then gradually dripped into the reactor. The mixture is then cooled to 20°C, and the crystallised product is filtered, to obtain 162 grams of the compound of Formula 1. The IR spectrum of the resulting product is shown in Figure 3.

### Example 4

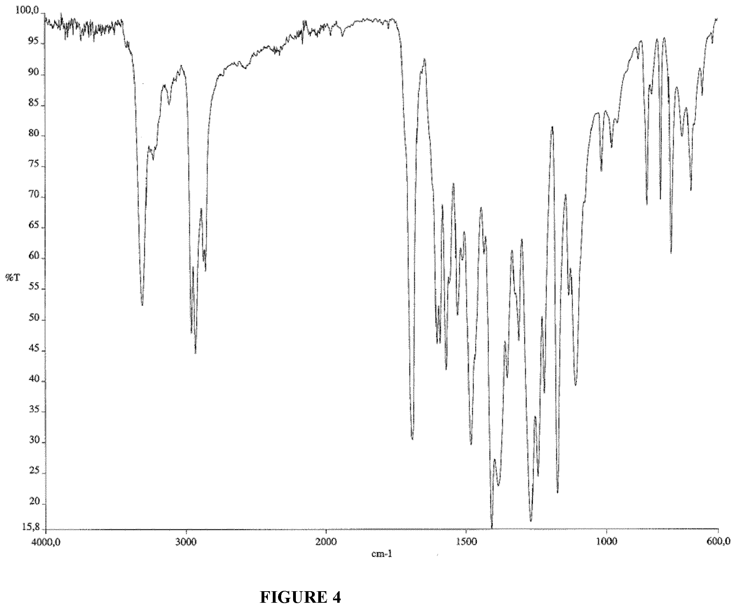

300 ml of methyl alcohol and the solid obtained in Example 2 are loaded into a laboratory reactor. The mixture is heated to boiling until completely dissolved. The mixture is then cooled to 20°C, and the crystallised product is filtered, to obtain 90 grams of the compound of Formula 1. The IR spectrum of the resulting product is shown in Figure 4.

## Claims

1. A process for the preparation of the compound of formula 1 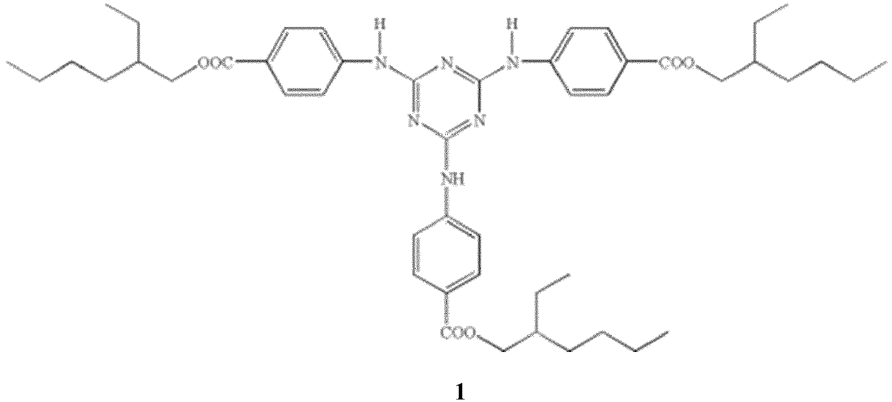 comprising crystallisation from methanol of the tautomer mixture obtained by reacting cyanuryl chloride with 2-ethylhexyl p-aminobenzoate.

2. Process according to claim 1 wherein the weight ratio of the tautomer mixture to methanol is 25% to 35%.

3. Process according to claim 1 or 2 wherein the crystallisation is carried out by cooling to 15°C the mixture solution heated to the boiling point of methanol.

4. Process according to claim 1 wherein the tautomer mixture is added to methanol in solid form.

5. Process according to claim 1 wherein the tautomer mixture is added to methanol in the molten state.

6. Product of formula 1 obtained by the process according to claims 1 to 5.
